# EUROPÄISCHE PATENTSCHRIFT

(11) **EP 0 768 294 B1**
(45) Veröffentlichungstag und Bekanntmachung des Hinweises auf die Patenterteilung: **12.09.2001**
(21) Anmeldenummer: 96115890.4
(22) Anmeldetag: 04.10.1996
(51) Int. Cl.: C07C 45/61, C07C 49/203, A61K 7/46

(54) **Verfahren zur Herstellung von 2,4,4,7-Tetramethyl-oct-6-en-3-on und dessen Verwendung als Riechstoff**
Process for preparing 2,4,4,7-tetramethyl-oct-6-en-3-on and its use as perfume
Procédé pour la préparation de 2,4,4,7-tetraméthyle-oct-6-ène-3-one et son utilisation comme parfum

(30) Priorität: 16.10.1995 DE 19539625
(43) Veröffentlichungstag der Anmeldung: 16.04.1997
(73) Patentinhaber: HAARMANN & REIMER GMBH, D-37601 Holzminden (DE)
(72) Erfinder: Dilk, Erich, Dr., 37603 Holzminden (DE); Wörner, Peter, 37603 Holzminden (DE)
(74) Vertreter: Petrovicki, Wolfgang, Dr.

(56) Entgegenhaltungen:
- US-A- 3 668 255
- CHEMICAL ABSTRACTS, vol. 49, no. 1, 1955 Columbus, Ohio, US; abstract no. 10879, XP002021344 & ZHUR. OBSHCHEI. KHIM. , Bd. 24, 1954, Seiten 1887-1893, A. T. BABAYAN ET AL.: "Alkylation in aqueous medium in the presence of quaternary ammonium salts."
- TETRAHEDRON LETTERS, Bd. 51, 1979, Seiten 4971-4974, XP002021343 M. T. REETZ ET AL.: "Lewis acid mediated alpha-alkylation of ketones using SN-1 reactive alkylating agents"

## Beschreibung

Die Erfindung betrifft die Verwendung von 2,4,4,7-Tetramethyl-oct-6-en-3-on (nachfolgend "TMO") zur Herstellung von Riechstoffkompositionen und ein 2-Phasen-Verfahren zu seiner Herstellung.

Aus der US-PS 3 668 255 ist bekannt, daß offenkettige verzweigte olefinisch ungesättigte Verbindungen einen angenehmen Geruch besitzen, so daß sie sich für die Formulierung von Parfums und Parfumbasen eignen. Eine der dort genannten Verbindungen ist TMO.

TMO kann nach M.T. Reetz et al. (Tetrahedron Letters 51, (1979) 4971) durch Alkylierung des Silenolethers von Diisopropylketon mit Prenylchlorid (= 1-Chlor-3-methyl-2-buten) oder gemäß US-PS 3 668 255 durch Prenylierung von Diisopropylketon unter Verwendung von festem Alkalihydroxid in Gegenwart von Ammoniak oder einem Amin hergestellt werden.

Eine konkrete Vorschrift für die Herstellung von TMO enthält die US-PS 3 668 255 allerdings nicht. Bei der Übertragung der allgemein empfohlenen Reaktionsbedingungen auf die TMO-Herstellung stellt sich dann heraus, daß TMO nur in Spuren entsteht. Woran dies liegt, konnten wir nicht feststellen; allerdings fällt auf, daß die Ausgangsverbindung Diisopropylketon in der US-PS 3 668 255 nicht genannt wird, obwohl die Aufzählung der Ketone sehr umfangreich ist. Bei näherer Betrachtung fällt allerdings auf, daß als Ausgangskomponenten nur solche Ketone empfohlen werden, die in mindestens einer α-Stellung keine Verzweigungsstelle besitzen. Möglicherweise haben die Autoren bereits selbst erkannt, daß das empfohlene Verfahren nicht auf alle beliebige Ketone mit Erfolg anwendbar ist.

Überraschenderweise wurde nun gefunden, daß die Prenylierung von Diisopropylketon zu guten Ausbeuten an TMO führt, wenn die Reaktion in Gegenwart von wäßriger Phase und eines Phasentransferkatalysators durchgeführt wird.

Gegenstand der Erfindung ist also ein Verfahren zur Herstellung von 2,4,4,7-Tetramethyl-oct-6-en-3-on durch Umsetzung von Diisopropylketon und 1-Chlor-3-methyl-2-buten,

dadurch gekennzeichnet, daß die Umsetzung in einem 2-Phasensystem enthaltend eine wäßrige Phase in Gegenwart eines Phasentransferkatalysators durchgeführt wird. Das Diisopropylketon kann in Mengen von 0,5 bis 10, vorzugsweise 0,8 bis 2 mol

pro mol Prenylchlorid eingesetzt werden.

Die Phasentransferkatalysatoren für das erfindungsgemäße Verfahren umfassen Phosphonium-, Sulfonium- und - vorzugsweise - Ammoniumverbindungen. Bevorzugte Ammoniumverbindungen entsprechen der Formel (I)

R¹R²R³R⁴ N^{⊕}X^{⊖} (I)

worin
- R¹ bis R⁴: unabhängig voneinander C₁-C₁₈-Alkyl oder Benzyl und
- X^{⊖}: Iodid, Bromid, Chlorid, Hydroxid oder Hydrogensulfat
bedeuten. Bevorzugte Verbindungen (I) umfassen insbesondere die Tetrabutylammoniumsalze.

Die Verbindungen (I) können in Mengen von 0,1 bis 10, vorzugsweise 1 bis 5 mol-%, bezogen auf die im Unterschuß eingesetzte Ausgangsverbindung, verwendet werden.

Die Reaktion wird im allgemeinen in Gegenwart einer Base durchgeführt. Geeignete Basen sind vor allem die Alkalimetallhydroxide, wie Natrium- und Kaliumhydroxid. Bevorzugte Mengen sind 1 bis 10 mol pro mol Prenylchlorid. Nach einer bevorzugten Ausführungsform besteht die wäßrige Phase aus einer gesättigten Lösung von Alkalihydroxid.

Die Reaktion kann mit oder ohne organische, mit Wasser nicht-mischbare Lösungsmittel durchgeführt werden; auf wassermischbare organische Lösungsmittel wie Methanol, Ethanol, Aceton etc. wird vorzugsweise verzichtet. Geeignete organische Lösungsmittel umfassen beispielsweise aliphatische Kohlenwasserstoffe wie Petrolether, cycloaliphatische Kohlenwasserstoffe wie Cyclohexan, Aromaten wie Benzol, Toluol und Xylol. Bei Verwendung solcher Lösungsmittel kann die Menge, bezogen auf die Summe der Ausgangskomponenten, 50 bis 300, vorzugsweise 80 bis 200 Gew.-% betragen.

Die wäßrige Phase kann innerhalb breiter Mengenbereiche variieren. Bevorzugte Mengen sind 80 bis 500, vorzugsweise 100 bis 300 Gew.-%, bezogen auf die Summe der Ausgangskomponenten.

Das erfindungsgemäße Verfahren kann bei Temperaturen zwischen 20 und 90°C, vorzugsweise 30 bis 80°C durchgeführt werden. Die Reaktionszeiten können 1 bis 25 Stunden, vorzugsweise 4 bis 16 Stunden betragen.

Zur Aufarbeitung kann man die Phasen trennen und das Reaktionsprodukt aus der organischen Phase, beispielsweise durch Destillation, gegebenenfalls unter vermindertem Druck, gewinnen.

Das erfindungsgemäße Verfahren liefert das TMO in guten Ausbeuten. Dies ist umso erstaunlicher, als gemäß US-PS 3 668 255 wasserfreie Bedingungen bevorzugt werden (Sp. 5, Z. 47 bis 50). Es hat also den Anschein, als wenn für die Herstellung von TMO ein dem Vorschlag von Babayan et al., Zhur Obshschei Khim 24 (1954), 1887; Chem. Abst. 49 (1955), 10 879 analoges Verfahren gerade mit Diisopropylketon sehr zufriedenstellend verläuft, obwohl die Autoren der US-PS 3 668 255 für die von ihnen verwendeten Ausgangsketone offenbar gravierende Nachteile festgestellt haben.

TMO läßt sich gut mit anderen Riechstoffen zu neuartigen interessanten Kompositionen kombinieren, wobei die Menge TMO vorzugsweise 1 bis 50 Gew.-%, bezogen auf die gesamte Komposition beträgt. Überraschenderweise wurde gefunden, daß die geruchlichen Aspekte des TMO insbesondere in Kombination mit anderen Riechstoffmaterialien verstärkt hervortreten bzw. zu neuen Geruchsfacetten beitragen.

So zeichnet sich Tetramethyloctenon durch eine spezielle Wirkung im herb-frischen Duftbereich aus. Hierbei werden insbesondere Geruchseindrücke aus dem Duftfeld von Bergamotte, Grapefruit und Muskatellersalbeiöl hervorgerufen.

Es eignet sich deshalb hervorragend für Duftkompositionen, wie z.B.
a) herbe Eau de Toilette-Noten, in denen das TMO einen bitteren Bergamotteeindruck vermittelt, der bis zum markant, herb-männlichen Dufteinruck von Muskatellersalbeiöl reicht,
b) grapefruithafte Kosmetikdüfte, in denen das TMO interessante Frischeeffekte in der Kopfnote hervorruft und Impact verleiht,
c) Nachbildungen von ätherischen Ölen, wo es den Charakter von Muskatellersalbeiöl ausgezeichnet unterstreicht.

Weiterer Gegenstand der Erfindung ist daher die Verwendung von TMO in Parfümkompositionen, die einen herb-frischen Charakter aufweisen.

Außer in der Feinparfümerie können derartige Kompositionen zur Parfumierung von Kosmetika wie Cremes, Lotionen, Aerosolen, Toilettenseifen, technischen Artikeln wie Reinigungs- und Waschmitteln, Weichspülern, Desinfektionsmitteln und Textilbehandlungsmitteln dienen.

Die Prozentangaben der nachfolgenden Beispiele beziehen sich, sofern nicht anders angegeben, auf das Gewicht.

### Beispiele

### Beispiel 1

### 2,4,4,7-Tetramethyl-oct-6-en-3-on

In einem 4 l-Doppelmantelgefäß werden 2400 g Natronlauge (50 %ig), 40 g Tetrabutylammoniumbromid und 548 g Diisopropylketon vorgelegt und auf 30°C erwärmt. Unter Rühren werden 418 g Prenylchlorid innerhalb 1 Stunde zudosiert und anschließend 16 Stunden nachgerührt. Man fügt 1200 g Wasser hinzu, rührt 20 Minuten und trennt anschließend die Phasen. Man erhält 895 g organische Phase, die über eine 60 cm Füllkörperkolonne destilliert wird.

Es werden 437 g (60 % d. Th., bezogen auf eingesetztes Prenylchlorid) Produkt Kp_{10mbar} = 112°C, erhalten.
Geruch: Grapefruit, Vetiver, Muskatellersalbei, Bergamotte, guter Impact

### Anwendung

### Anwendungsbeispiel 1:

| | A Gew.-Teile | B Gew.-Teile |
|---|---|---|
| Bergamott Identoil farblos ® H+R | 175 | 175 |
| Dihydromyrcenol | 200 | 200 |
| Citronenöl | 100 | 100 |
| Vertocitral ® H+R | 5 | 5 |
| Methyldihydrojasmonat ® Firmenich | 50 | 50 |
| Citrophoral Supra ® H+R | 5 | 5 |
| Lavandinoel Grosso | 25 | 25 |
| Muskateller Salbeiöl Franz. | 10 | 10 |
| Geranium Chin. Synthessence ® H+R | 30 | 30 |
| Damascon Betra ® Firmenich | 1 | 1 |
| Precyclemone B ® IFF | 19 | 19 |
| Isocyanat ® H+R | 15 | 15 |
| Sandolen H+R | 5 | 5 |
| Cedrylketon | 100 | 100 |
| Chromanolid 50 % in DEP ® H+R | 100 | 100 |
| Evernyl ® Givaudan-Roure | 10 | 10 |
| Ambroxid Rein ® H+R | 10 | 10 |
| Dipropylenglykol | 140 | 100 |
| 2,4,4,7-Tetramethyl-oct-6-en-3-on | - | 40 |

Durch den Zusatz von Tetramethyloctenon wird eine unverkennbare frisch-grüne Spitze in der Kopfnote der herben Herrenkomposition bewirkt.

### Anwendungsbeispiel 2:

| | A Gew.-Teile | B Gew.-Teile |
|---|---|---|
| Aldehyd C10 | 2,0 | 2,0 |
| Aldehyd C11 Undecylen | 1,0 | 1,0 |
| Aldehyd C12 MNA | 2,0 | 2,0 |
| Vertocitral ® H+R | 5,0 | 5,0 |
| Isoananat ® H+R | 5,0 | 5,0 |
| Dihydromyrcenol ® IFF | 75,0 | 75,0 |
| Terpinylacetat | 50,0 | 50,0 |
| Citral IFRA ® H+R | 18,0 | 18,0 |
| Geranitril ® BASF | 5,0 | 5,0 |
| Orangenoel Weiss | 120,0 | 120,0 |
| Lavandinoel Grosso | 20,0 | 20,0 |
| Krauseminzoel Amerik. | 2,0 | 2,0 |
| Isobornylacetat | 40,0 | 40,0 |
| Borneol L | 5,0 | 5,0 |
| Hydroxybenzylaceton Para | 1,0 | 1,0 |
| Lilial | 5,0 | 5,0 |
| Lyral ® IFF | 10,0 | 10,0 |
| Linalool | 50,0 | 50,0 |
| Dimethylbenzylcarbinylbutyrat | 2,0 | 2,0 |
| Rosenoxid Rac. ® Dragoco | 1,0 | 1,0 |
| Damascon Alpha 10 % DEP ® Firmenich | 3,0 | 3,0 |
| Benzylacetat | 10,0 | 10,0 |
| Jasmin 151 ® H+R | 5,0 | 5,0 |
| Benzylsalicylat | 124,0 | 124,0 |
| Hexylsalicylat | 30,0 | 30,0 |
| Anethol NPU 21/22 Grad C | 2,0 | 2,0 |
| Cumarin | 5,0 | 5,0 |
| Cedrenylacetat IFF ® IFF | 15,0 | 15,0 |
| Patchoulyoel Entf. H+R ® H+R | 10,0 | 10,0 |
| Piconia ® IFF | 10,0 | 10,0 |
| Sandel H+R ® H+R | 5,0 | 5,0 |
| Evernyl ® Givaudan-Roure | 5,0 | 5,0 |
| Juchtenoel Hell 10 % DEP | 2,0 | 2,0 |
| Cuminaldehyd | 2,0 | 2,0 |
| Ambroxid 30 % in Hercolyn 10 % DEP ® H+R | 3,0 | 3,0 |
| Niso-Salicylat 38/62 ® H+R | 20,0 | 20,0 |
| DEP | 30 | - |
| 2,4,4,7-Tetramethyl-oct-6-en-3-on | - | 30 |
| DEP = Diethylphthalat | | |

Die Mischung riecht nach Zugabe von 2,4,4,7-Tetramethyl-oct-6-en-3-on lebhafter frisch und deutlich spritziger nach Grapefruit.

### Anwendungsbeispiel 3:

| | A / Gew.-Teile | B / Gew.-Teile |
|---|---|---|
| Nonal | 1,0 | 1,0 |
| Undecanol | 1,0 | 1,0 |
| Linalylacetat | 645,0 | 645,0 |
| Linalylisobutyrat | 20,0 | 20,0 |
| Terpinylacetat Alpha L | 5,0 | 5,0 |
| Terpinolen | 2,0 | 2,0 |
| Pinen Beta Supra | 8,0 | 8,0 |
| Limonen L -80 Grad | 10,0 | 10,0 |
| Pinen Alpha Supra | 4,0 | 4,0 |
| Ocimen | 3,0 | 3,0 |
| Eucalyptol | 2,0 | 2,0 |
| Cymol Para Supra | 6,0 | 6,0 |
| Bornylacetat L Krist. | 2,0 | 2,0 |
| Camphen L Supra | 2,0 | 2,0 |
| Nerolidol | 5,0 | 5,0 |
| Linalooloxid | 3,0 | 3,0 |
| Linalool | 80,0 | 80,0 |
| Terpineol Alpha L Krist. | 10,0 | 10,0 |
| Nerol Supra | 20,0 | 20,0 |
| Geraniol Supra | 40,0 | 40,0 |
| Geranylacetat Supra | 20,0 | 20,0 |
| Geranylisobutyrat | 10,0 | 10,0 |
| Nerylacetat Supra | 10,0 | 10,0 |
| Nonadienal/A in TEC | 1,0 | 1,0 |
| Caryophyllen Rekt. | 30,0 | 30,0 |
| Flouveöl | 1,0 | 1,0 |
| Muskateller Salbei Abs. | 7,0 | 7,0 |
| Liatrix Abs. Incol. Maximarome | 1,0 | 1,0 |
| Ambroxid Rein ® (H+R) | 1,0 | 1,0 |
| Triethylcitrat1,0 | 50,0 | - |
| 2,4,4,7-Tetramethyl-oct-6-en-3-on | - | 50,0 |

Durch den Ersatz von Triethylcitrat durch 2,4,4,7-Tetramethyl-oct-6-en-3-on wird der Eindruck von echtem Muskatellersalbeiöl deutlich in einem Umfang verbessert, der durch die Zugabe von 5 % nicht zu erwarten war.

## Patentansprüche

1. Riechstoffkompositionen mit herbfrischem Charakter, enthaltend 2,4,4,7-Tetramethyl-oct-6-en-3-on.

2. Riechstoffkomposition, enthaltend 1 bis 50 Gew.-% 2,4,4,7-Tetramethyl-oct-6-en-3-on, bezogen auf die gesamte Komposition.

3. Verwendung von 2,4,4,7-Tetramethyl-oct-6-en-3-on in Riechstoffkompositionen mit herbfrischem Charakter.

4. Verfahren zur Herstellung von 2,4,4,7-Tetramethyl-oct-6-en-3-on durch Umsetzung von Diisoproylketon und 1-Chlor-3-methyl-2-buten, **dadurch gekennzeichnet, daß** die Umsetzung in Gegenwart von wäßrigem Alkalihydroxid im Zweiphasensystem und in Gegenwart einer Phosphonium-, Sulfonium- oder Ammoniumverbindung als Phasentransferkatalysator durchgeführt wird.

5. Verfahren nach Anspruch 4, wonach pro Mol 1-Chlor-3-methyl-2-buten 0,5 bis 10 Mol Diisopropylketon eingesetzt werden.

6. Verfahren nach Anspruch 4, wonach der Phasentransferkatalysator aus der Gruppe der quarternären Ammoniumsalze ausgewählt wird.

7. Verfahren nach Anspruch 4, wonach 0,1 bis 10 mol.-% Phasentransferkatalysator, bezogen auf die im Unterschuß eingesetzte Ausgangsverbindung, eingesetzt werden kann.

## Claims

1. Fragrance compositions having a fresh/tangy nature containing 2,4,4,7-tetramethyloct-6-en-3-one.

2. Fragrance composition containing 1 to 50 wt.% of 2,4,4,7-tetramethyloct-6-en-3-one, relative to the entire composition.

3. Use of 2,4,4,7-tetramethyloct-6-en-3-one in fragrance compositions having a fresh/tangy nature.

4. Process for the production of 2,4,4,7-tetramethyl-oct-6-en-3-one by reaction of diisopropyl ketone and 1-chloro-3-methyl-2-butene, **characterised in that** the reaction is performed in the presence of aqueous alkali metal hydroxide in a two-phase system and in the presence of a phosphonium, sulfonium or ammonium compound as phase-transfer catalyst.

5. Process according to claim 4, in accordance with which 0.5 to 10 mol of diisopropyl ketone are used per mol of 1-chloro-3-methyl-2-butene.

6. Process according to claim 4, in accordance with which the phase-transfer catalyst is selected from among the group of quaternary ammonium salts.

7. Process according to claim 4, in accordance with which 0.1 to 10 mol% of phase-transfer catalyst may be used relative to the starting compound which is used in a substoichiometric quantity.

## Revendications

1. Compositions de parfums à caractère frais-vert, contenant de la 2,4,4-7-tétraméthyl-oct-6-én-3-one.

2. Compositions de parfums contenant 1 à 50 % en poids de la 2,4,4-7-tétraméthyl-oct-6-én-3-one sur son poids total.

3. Utilisation de la 2,4,4-7-tétraméthyl-oct-6-én-3-one dans des compositions de parfums à caractère frais-vert.

4. Procédé pour la préparation de la 2,4,4-7-tétraméthyl-oct-6-én-3-one par réaction de la diisopropylcétone et du 1-chloro-3-méthyl-2-butène, **caractérisé en ce que** la réaction est réalisée en présence d'un hydroxyde alcalin aqueux dans un système à deux phases et en présence d'un dérivé de phosphonium, de sulfonium ou d'ammonium qui sert de catalyseur à transfert de phase.

5. Procédé selon la revendication 4, dans lequel on utilise 0,5 à 10 mol de diisopropylcétone par mole du 1-chloro-3-méthyl-2-butène.

6. Procédé selon la revendication 4, dans lequel le catalyseur à transfert de phase est choisi dans le groupe des sels d'ammonium quaternaire.

7. Procédé selon la revendication 4, dans lequel on peut utiliser de 0,1 à 10 mol % du catalyseur à transfert de phase par rapport au composé de départ mis en oeuvre en défaut.
